# FASCICULE DE BREVET EUROPEEN

(11) **EP 0 799 889 B1**
(45) Date de publication et mention de la délivrance du brevet: **11.02.2004**
(21) Numéro de dépôt: 96401137.3
(22) Date de dépôt: 24.05.1996
(51) Int. Cl.: C12N 15/12, C07K 14/705, C12N 5/10, A61K 38/17, G01N 33/53

(54) **Famille de canaux potassium de mammifère, leur clonage et leur utilisation notamment pour le criblage de drogues**
Säugtieren Kalikanalfamilie, deren Klonierung und Anwendung für Drogenscreening
Mammalian potassium channel family, their cloning and their use for drug screening

(30) Priorité: 08.02.1996 FR 9601565
(43) Date de publication de la demande: 08.10.1997
(73) Titulaire: CENTRE NATIONAL DE LA RECHERCHE SCIENTIFIQUE (CNRS), 75016 Paris (FR)
(72) Inventeur: Lesage, Florian, 06100 Nice (FR); Guillemare, Eric, 06100 Nice (FR); Fink, Michel, 06150 Cannes la Bocca (FR); Duprat, Fabrice, 06220 Vallauris (FR); Lazdunski, Michel, 06100 Nice (FR); Romey, Georges, 06200 Nice (FR); Barhanin, Jacques, 06100 Nice (FR)
(74) Mandataire: Pernez, Helga

(56) Documents cités:
- WO-A-94/28131
- WO-A-95/21943
- NATURE, AUG 24 1995, 376 (6542) P690-5, ENGLAND, XP002017567 KETCHUM KA ET AL: "A new family of outwardly rectifying potassium channel proteins with two pore domains in tandem."
- NATURE, SEP 15 1994, 371 (6494) P243-6, ENGLAND, XP002017568 LU Z ET AL: "Electrostatic tuning of Mg2+ affinity in an inward-rectifier K+ channel."
- EMBO (EUROPEAN MOLECULAR BIOLOGY ORGANIZATION) JOURNAL, 15 (5). 1996. 1004-1011., XP002017569 LESAGE F ET AL: "TWIK-1, a ubiquitous human weakly inward rectifying K+ channel with a novel structure"
- GENOMICS, 34 (1). 1996. 153-155., XP000609245 LESAGE F ET AL: "Assignment of the human weak inward rectifier K+ channel TWIK-1 gene to chromosome 1q42-q43"

## Description

La présente invention concerne une nouvelle famille de canaux potassium. Elle concerne plus particulièrement le clonage d'un canal potassium humain qui constitue le premier membre d'une nouveau groupe fonctionnel et structurel de canaux potassium. L'abondance de ce canal et sa présence dans un grand nombre de tissus sont de nature à lui confèrer un rôle primordial dans le transport du potassium chez un grand nombre de types cellulaires.

Les canaux potassium sont ubiquitaires chez les cellules eucaryotes et procaryotes. Leur exceptionnelle diversité fonctionnelle en font des candidats idéals pour un grand nombre de processus biologiques dans les cellules vivantes (Rudy, B., 1988, *Neurosciences,* 25, 729-749 ; Hille, B., 1992, "*Ionic Channels of Excitable Membrane*", 2nd edn, Sinauer, Sunderland, Massachussetts). Chez les cellules excitables, les canaux K⁺ définissent la forme des potentiels d'action et la fréquence de l'activité électrique, et joue un role majeur dans l'intégration neuronale, la contraction musculaire ou la sécrétion hormonale. Chez les cellules non-excitables, leur expression semble être corrélée à des stades spécifiques du développement de la cellule (Barres, B. A. et al., 1990, *Annu. Rev. Neurosci.,* 13, 441-474). Chez la plupart des cellules, des types particuliers de canaux K⁺ jouent un rôle vital pour déterminer le potentiel électrique de la membrane au repos en réglant la perméabilité membranaire aux ions K⁺. Ces canaux présentent la particularité d'être instantanés et ouverts dans un large gamme de potentiels membranaires.

Des travaux de clonage récents ont permis d'identifier un grand nombre de sous-unités capables de former de canaux potassium (Betz, H., 1990, *Biochemistry*, 29, 3591-3599 ; Pngs, O., 1992, *Physiol. Rev.,* 72, S69-88 ; Salkoff, L. et al., 1992, *Trends Neurosci.,* 15, 161-166 ; Jan, L. Y. and Y. N. Jan, 1994, Nature, 371, 119-122 ; Doupnik, C. A. et al., 1995, *Curr. Opin. Neurobiol*., 5, 268-277) qui pourraient être régulés par d'autres types de sous-unités (Aldrich, R. W., 1994, *Curr. Biol*., 4, 839-840 ; Isom, L. L. et al., 1994, *Neuron,* 12, 1183-1194 ; Rettig, J. et al., 1994, *Nature,* 369, 289-294 ; Attali, B. et al., 1995, *Proc. Natl. Acad. Sci. USA*, 92, 6092-6096).

Les sous-unités des canaux K⁺ voltage-dépendants activés par dépolarisation (familles Kv) et des canaux K⁺ calcium-dépendant présentent six domaines transmembranaires hydrophobes, dont l'un (S4) contient des charges positives répétées qui confèrent à ces canaux leur sensibilité au voltage et en conséquence dans leur rectification sortante fonctionnelle (Logothetis, D. E. et al., 1992, *Neuron,* 8, 531-540 ; Bezanilla, F. et Stefani, E., 1994, *Annu. Rev. Biophys. Biomol. Struct*., 23, 819-846).

Les canaux K⁺ avec une rectification entrante (Familles Kir) ont seulement deux domaines transmembranaires. Ils ne possèdent pas le segment S4 et la rectification entrante résulte d'un blocage voltage-dépendant par le magnésium cytoplasmique (Matsuda, H, 1991, *Annu. Rev. Physiol*., 53, 289-298 ; Lu, Z. et Mackinnon, R., 1994, *Nature,* 371, 243-246 ; Nichols, C. G. eta;., 1994, *J. Physiol. London,* 476, 399-409).

Un motif structural commun, dénommé domaine P, est rencontré dans les deux groupes, et constitue un élement essentiel de la structure du pore perméable au K⁺. La présence de ce motif dans une protéine membranaire est considéré comme la signature de la structure d'un canal K⁺ (Pongs, O., 1993, *J. Membrane Biol*., 136, 1-8 ; Heginbotham, L. et al., 1994, *Biophys. J.,* 66, 1061-1067 ; Mackinnon, R. 1995, *Neuron*, 14, 889-892 ; Pascual, J. M. et al., 1995, *Neuron,* 14, 1055-1063).

La présente invention est fondé sur le clonage d'un canal K⁺ qui est le premier membre d'une nouveau groupe structurel et fonctionnel de canaux potassium. Ce nouveau canal K⁺ a une nouvelle architecture moléculaire avec quatre segments trans-membranaire et deux domaine P. D'un point de vue fonctionnel, ce canal est remarquable en ce qu'il présente des propriétés de rectification entrante peu marquées. Ce nouveau canal est dénommé, dans ce qui suit, TWIK-1, pour désigner la terminologie anglaise "Tandem of P domains in a Weak Inward rectifying K⁺ channel". Son abondance et sa présence dans un grand nombre de tissus sont de nature à lui confèrer un rôle primordial dans le transport du potassium chez un grand nombre de types cellulaires.

La mise en évidence de cette nouvelle famille de canaux potassium et le clonage d'un membre de cette famille permet notamment de disposer de nouveaux moyens de criblages de drogues capables de moduler l'activité de ces nouveaux canaux potassium et donc de prévenir ou traiter des maladies impliquant ces canaux.

Le travail de recherche ayant mené au clonage du canal TWIK-1 a été conduit de la manière décrite ci-après, où il sera fait référence aux séquences et dessins en annexe dans lesquels :
- SEQ ID NO : 1 représente la séquence la séquence nucléotidique de l'ADNc de TWIK-1 et la séquences en acides aminés de la séquence codante.
- SEQ ID NO : 2 représente la séquence en acides aminés de la protéine TWIK-1.

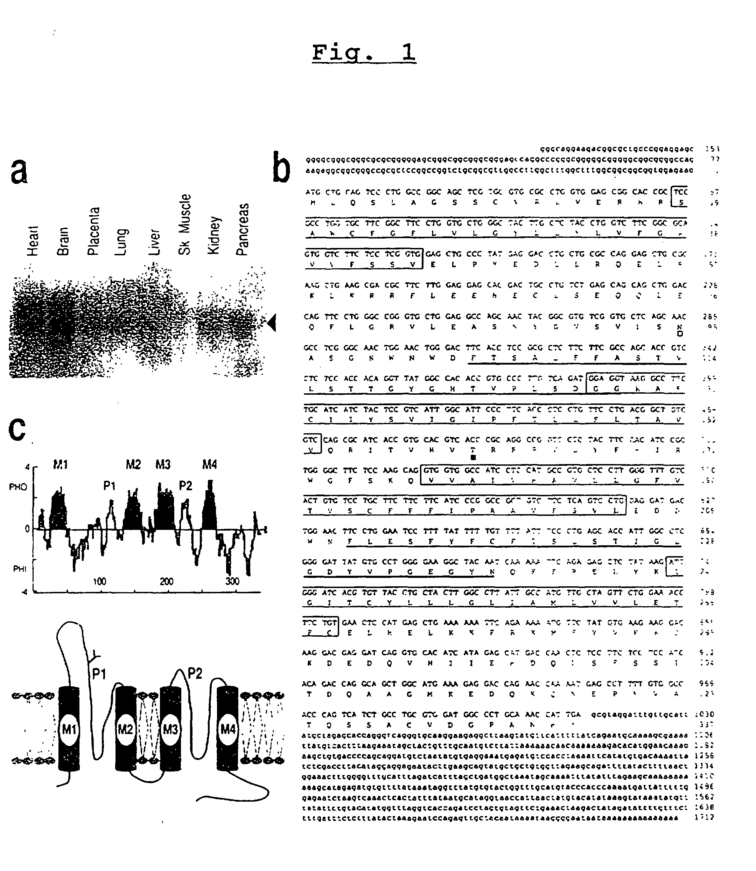

- La figure 1 représente l'analyse en Northern blot, les séquences en nucléotides et la séquence déduite en acides aminés, ainsi que le profile d'hydrophobicité de TWIK-1. (a) : expression de l'ARNm de TWIK-1 dans des tissus humains; chaque voie contient 5 µg de poly(A)⁺; l'autoradiogramme a été exposé 24 heures. (b) : séquence de l'ADNc de TWIK-1 et la séquences en acides aminés de la séquence codante. Les segments supposés trans-membranaires sont encadrés et les domaines P sont soulignés; o, représente un site potentiel de glycosilation, et ■ représente le résidu thréonine dans lesite consensus de reconnaissance de la protéine kinase C. (c) : l'analyse d'hydrophobicité et la topologie de TWIK-1 qui en est déduite; les valeurs d'hydrophobicité ont été calculées conformément à la méthode de Kyte et Doolittle (taille de la fenêtre de 11 acides aminés) et sont rapportées vis-à-vis de la position de l'acide aminé; les pics hydrophobes ombrés correspondent aux segments trans-membranaires.

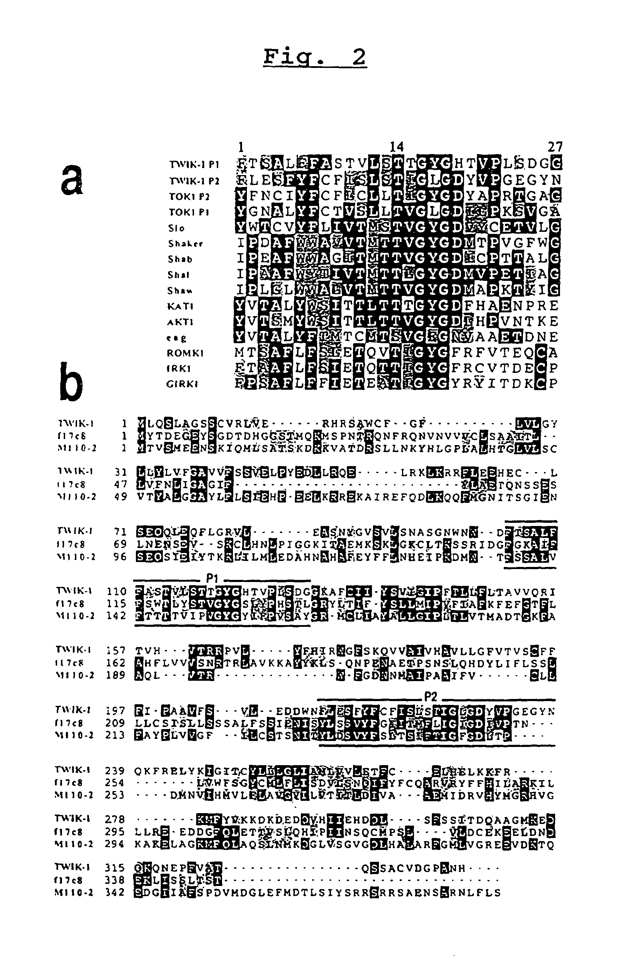

- La figure 2 représente les alignements de séquences. (a) : alignement des domaines P de TWIK-1, TOC/YORK, et d'autres familles de canaux K+ représentatives; les résidus identiques et conservés sont encadrés respectivementen noir et en gris. (b) : alignement de TWIK-1 avec des homologues potentiels de *C. elegans;* les séquences M110.2 et F17C8.5 ont été déduites des séquences des gènes (numéros d'accès respectifs Z49968 et Z35719); l'épissage informatisé des autres séquences génomique de *C. elegans* (numéros d'accès Z49889, P34411 et Z22180) n'est pas suffisament précis pour permettre leur alignement parfait et n'est donc pas représenté.

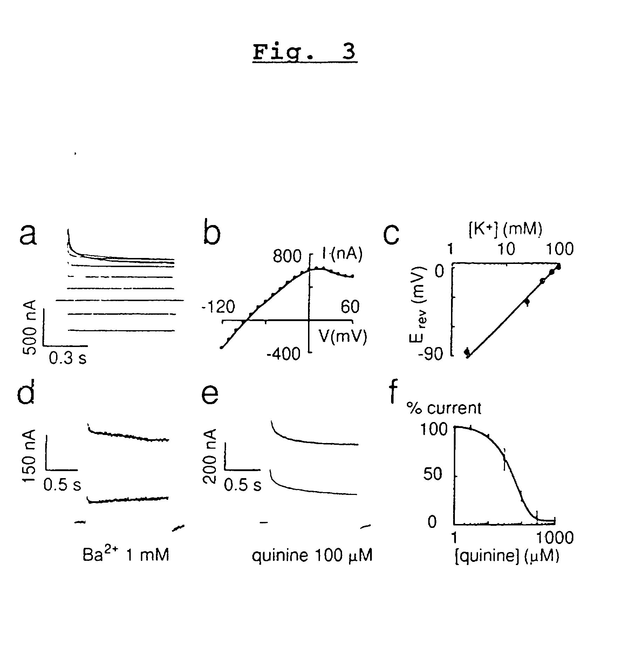

- La figure 3 montre les propriétés biophysiques et pharmacologiques de courants K⁺ enregistrés par la technique de voltage-imposé sur des oocytes de Xenope ayant reçus une injection d'ARNc de TWIK-1; (a) : l'oocyte a été maintenu à un potentiel de maintien (HP) de -80 mV et les courants ont été enregistrés à la suite de sauts de voltage de 1-s de -120 à + 60 mV par incrément de 20 mV. (b) : relation régulièrecourant-voltage, selon la même expérience qu'en (a). (c) : renversement de potentiel des courants de TWIK-1 (Eᵣₑᵥ) en fonction de la concentration externe en K⁺. (d) : traces de courants liées à des dépolarisations de +30 mV à partir d'un potentiel maintenu (HP) de -80 mV en l'absence (trace supérieur) et en présence (trace inférieur) de 1 mM de Ba²⁺. (e) : effet bloquant de 100 µM de quinine, même protocole qu'en (d). (f) : relation dose-réponse du blocage des courants de TWIK-1 par la quinine.

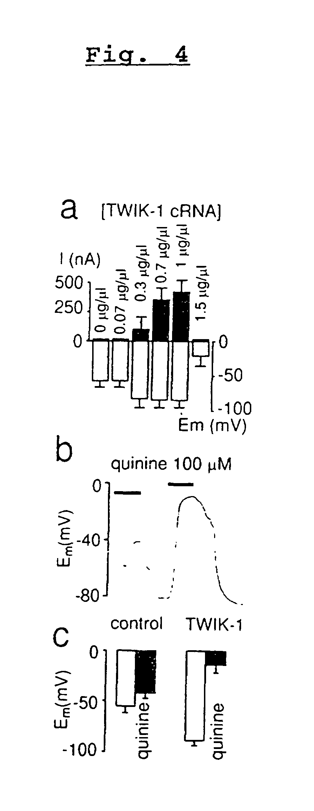

- La figure 4 montre l'influence de l'expression de TWIK-1 sur le potentiel membranaire. (a) : relations dose-réponse de l'ARNc; rangé du haut = état d'équilibre des courants sortants mesurés à +30 mV; rangée du bas = potentiels membranaires associés au repos. (b) : effet de 100 µM de quinine sur le potentiel membranaire d'un oocyte n'ayant pas reçu d'injection (trace de gauche) et d'un oocyte ayant reçu 20 ng d'ARNc de TWIK-1. (c) : traitement statistique des effets dépolarisants de 100 µM de quinine sur des oocytes n'ayant pas reçus d'injection (barres de gauche) et sur des oocytes ayant reçus une injection de 20 ng d'ARNc de TWIK-1 (barres de droite); contrôle (barre ouverte), + quinine (barres solides); chaque barre représente la moyenne ± SD de 5 oocytes.

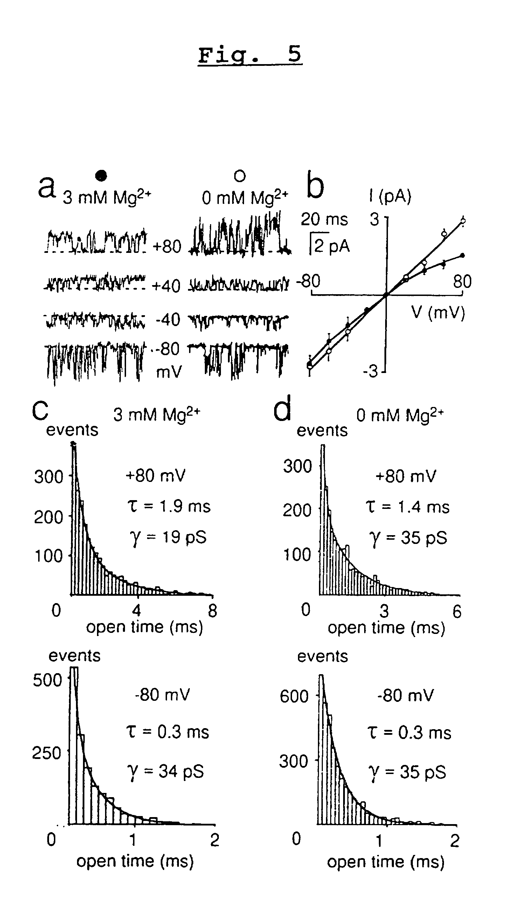

- La figure 5 montre les propriétés de de canal unique de TWIK-1. (a) : traces de courants enregistrées dans la configuration entrée-sortie aux potentiels de membrane indiqués en l'absence (m) ou en présence (•) de Mg²⁺ interne (3 mM) et en symétrique de 140 mM de K⁺. (b) : moyenne des courbes I-V (n = 10). (c et d) : temps ouvert de distribution obtenues à + 80 mV (histogrammes du haut) et à -80mV (histogrammes du bas) en présence of 3 mM Mg²⁺ (c) ou en l'absence de Mg²⁺ (d).

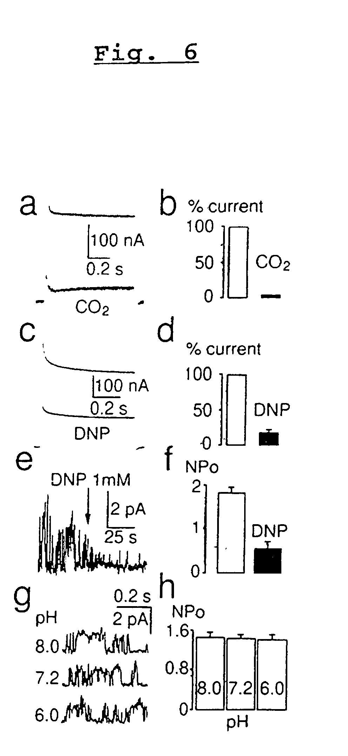

- La figure 6 montre le blocage des canaux TWIK-1 par le pH interne. (a et b) : effet bloquant de l'acidification interne sur les courants TWIK-1, induite par perfusion de CO₂; (a) : traces de courants superimposés provoqués par une phase de dépolarization à - 30 mV à partir de HP = -80 mV, contrôle (trace supérieure), effet quand l'éliquilibre est atteint en présence de CO₂ (trace inférieure); (b) : graphe (n = 5) montrant le bloquage presque complet des courants de TWIK-1 induit par du CO₂; (c et d) : acidification interne induite par l'application de DNP (1 mM). (c) : même protocole qu'en (a), contrôle (trace supérieure) et après 5 minutes d'application du DNP (trace inférieure); (d) : graphe (n = 4) indiquant le pourcentage de courant TWIK-1 restant après traitement par le DNP. (e et f) : voltage imposé (mode : patch attaché) dans des conditions symétriques de concentration en K⁺ (140 mM) maintenu à +80 mV. (e) déroulement dans le temps de l'effet de 1 mM de DNP (fléché) sur les activités de canal unique de TWIK-1. (f) : graphe (n = 4) montrant l'effet du DNP sur la probabilité moyenne d'ouverture NPₒ calculé pendant 1 minute d'enregistrement à partir de l'état d'équilibre. (g) : activités mesurées dans la configuration "patch-inside-out" à + 80 mV à différent pH interne. Graphe de barres (n = 10) de NPₒ en fonction du pH interne.

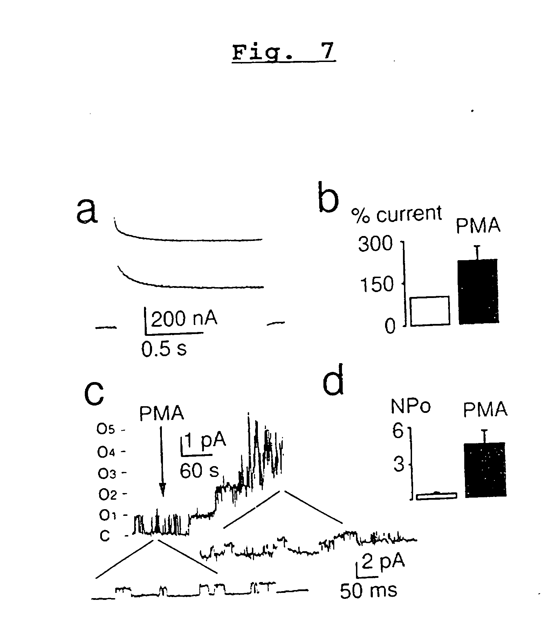

- La figure 7 montre l'activation des canaux TWIK-1 par le PMA, activateur de la protéine kinase C. (a) : perfusion pendant 10 minutes de PMA (30 nM) augmente le courant TWIK-1 (trace supérieure) provoqué par une phase de dépolarisation à +30 mV à partir de HP = -80 mV, courant de contrôle (trace inférieure). (b) graphe (n = 5) montrant l'effet d'activation du PMA sur les courants TWIK-1. (c et d) : configuration "patch" attaché en conditions de concentrations en K⁺ symétriques maintenu à +60 mV; (c) : déroulement dans le temps de l'effet de 30 nM de PMA sur les activités de canal unique; les enregistrements de l'activité du canal sont effectués avec un balayage rapide avant et après l'application du PMA; (d) : graphe de barres (n = 5) montrant l'effet d'activation du PMA sur NPₒ.

Les domaines P de canaux K⁺ ont été utilisés pour rechercher de séquences correspondantes dans la banque de données GenBank en mettant en oeuvre le programme d'alignement de séquence BLAST (Altschul, S. F., et al., 1990, *J. Mol. Biol*., 215, 403-410). Il a été ainsi identifié une séquence exprimée Tag humaine (EST, HSC3AH031) de 298 pb dont la séquence déduite en acides aminés comprend une séquence de domaine "P-like" non conventionnelle : GLG au lieu de GYG comme montré à la figure 2a. Il a été envisagé alors que cette séquence EST était une une copie partielle d'un ARNm codant un nouveau type de sous-unité de canal K⁺. Une sonde d'ADN a été préparée à partir de cette séquence afin de réaliser une bybridation avec un Northern blot (Clontech) de tissus multiples humains. Un transcrit de 1,9 kb a ainsi été abondamment trouvé, comme montre à la figure la, dans le coeur et le cerveau et, à un niveau moindre, dans le placenta, le poumon, le foi et le rein. La sonde d'ADN a été utilisée pour cribler une banque d'ADNc de rein et quatre clones independants ont été obtenus. les inserts d'ADNc de 1,8 à 1,9 kb de ces clones comportent tous le mêm cadré de lecture ouvert (ORF) contenant une régio identique à la séquence de 298 pb de HSC3AH031 et diffèrent seulement par la longueur de leurs séquences 5' non codantes.

### Structure primaire de TWIK-1.

Les caractéristiques suivantes ont été mise en évidence :
- Les séquences des clones d'ADNc contiennent un ORF de 1011 nucléotides codant pour un polypeptide de 336 acides aminés représenté à la figure 1b.
- Cette protéine comporte deux domaines P.
- En dehors des domaines P, aucun alignement significatif n'a été observé entre TWIK-1 et un canal K+ récemment cloné chez la levure et qui comporte également deux domaines P (Ketchum, K. A. et al, 1995, *Nature,* 376, 690-695).
- L'analyse d'hydrophobicité de TWIK-1, représenté à la figure 1c, révèle la présence de quatre domaines trans-membranaires, désignés T1 à T4.
- En plaçant l'extrémité NH2 sur la face cytoplasmique, conformément à l'abscence de peptide signal, on obtient le modèle de topologie représenté à la figure 1c.
- Dans ce modèle, les deux domaines P sont insérés dans la membrane depuis l'extérieur conformément à l'orientation connue de ces boucles dans les canaux K⁺.
- En outre, le motif structurel général de TWIK-1 est similaire au motif que l'on obtiendrait en faisant un tandem de deux sous-unités classiques rectifiant l'entrée d'un canal potassium. Comme un rectificateur entrant classique, TWIK-1 ne présente pas le segment hautement conservé S4 qui est responsable de la sensibilité au potentiel de membrane de la rectification entrante des canaux K+ de la famille Kv.
- Une large boucle non habituelle de 59 acides aminés est présente entre M1 et P1, de manière à étendre la longueur du linker M1-P1 du coté extracellulaire de la membrane.
- Un site de N-glycosilation potentiel est présent dans cette boucle.
- Trois sites consensus de phosphorylation sont présents aux extrémités N-terminale (Ser 19 pour la calcium calmodulin kinase II) et C-terminale (Ser 303 pour la caséine kinase II) des domaines cytoplasmiques, et dans le linker M2-M3 (Thrl61 pour la protéine kinase II).
- L'alignement des domaines P d'un groupe important de canaux K⁺ est donné à la figure 2a. Il montre que les régions constituant le pore sélectif au K⁺ sont bien conservées incluant le résidus G en position 16 et 18 et trois autres résidus indiquant des changements pratiquement exclusivement conservatifs aux positions 7, 14 et 17. De façon interessante, un résidu leucine est présent à la place d'une tyrosine conservée en position 17 dans le domaine P2 de TWIK-1, ou d'un phénylalanine en position 17 dans le domaine P du canal K⁺ de type eag.

### Les homologues de TWIK-1.

La comparaison de la séquence complète de TWIK-1 avec des séquences de la base de données Genbank a permis d'identifier au moins cing gènes de *Caenorhabditis elegans* qui ont été caractérisé dans le cadre du projet de Séquençage Nematode, et qui potentiellement code pour des homologues structuraux de TWIK-1. L'alignement de deux d'entre-eux avec TWIK-1 est représenté à la figure 2b. Les homologies de séquences totales entre les protéines déduites de *C. elegans* et TWIK-1 sont d'environ 55 à 60 % et environ 25 à 28 % d'identité. Les homologies entre séquences de *C. elegans* ne sont pas supérieures.

### Expression fonctionnelle de TWIK-1.

Pour l'étude fonctionnelle, la séquence codante de TWIK-1 a été inséré entre les séquences non-codantes 5' et 3' de *Xenopus* globin dans le vecteur pEXO (Lingueglia, E. et al., 1993, *J. Biol. Chem.,* 269, 13736-13739). Un ARN complémentaire (ARNc) a été transcrit de cette construction et injecté dans des oocytes de *X. laevis*. Un courant non-inactivant, absent des cellules non-injectées, a été mesuré par la technique de voltage imposé, comme représenté à la figure 3a. L'activation cinétique du courant est le plus souvent instantanée et ne peut pas être résolue car elle est masquée par la décharge capacitive du courant enregistré au début de l'impulsion. La relation courant-voltage est linéaire au-dessus de 0mV puis sature pour une dépolarisation plus forte de la membrane, comme montré à la figure 3b. TWIK-1 est donc K⁺ sélectif. Dans le cas d'une substitition du K⁺ externe par du Na⁺ ou par du N-méthyle D-gluconate, le renversement du potentiel des courants suit le potentiel d'équilibre K⁺ (E_{K}), comme représenté à la figure 3c. En outre, un changement par 10 de la concentration [(K)]ₒ conduit à un changement de 56 ± 2 mV de la valeur d'inversion du potentiel, conformément à l'équation de Nernst.

Comme montré à la figure 3, les courants K⁺ de TWIK-1 sont inhibés par Ba²⁺ (figure 3d) avec une valeur IC₅₀ de 100 µM, par la quinine (figure 3e et 3f) et par la quinidine (non représenté) avec des valeurs IC₅₀ respectivement de 50 et 95 µM. Les courants TWIK-1 sont légèrement sensible au TEA et au tedisamil anti-arythmique de classe III (30 % d'inhibition pour chacun, respectivement à 10 mM et 100 µM). Moins de 10 % d'inhibition ont été observés après application de 4-aminopyridine (1 mM), d'apamine (0,3 µM), de charybdotoxine (3 nM), de dedrotoxine (0, 1 µM), de clofilium (30 µM), d'amiodarone (100 µM) et de glibenclamide (30 µM). Le canal TWIK-1 n'est pas sensible aux ouvreurs de canal K+ que sont la cromakaline (100 µM) et le pinacidil (100 µM).

La figure 4 montre l'effet de l'augmentation des doses injectées d'ARNc de TWIK-1 sur l'expression indépendante du temps des courants K⁺ et sur le repos du potentiel membranaire (Eₘ). Dès que le courant apparaît, les oocytes deviennent davantage polarisés, pour atteindre une valeur de Eₘ proche de E_{K}. L'amplitude du courant de TWIK-1 atteint des valeurs de 0,6 à 0,8 µA pour l'injection de 20 ng par oocyte. Des doses supérieures d'ARNc de TWIK-1 sont toxiques consuisant à une baisse de l'expression. Dans les oocytes ayant reçus 20 ng d'ARNc, la quinine qui est le meilleur bloquant de TWIK-1, induit une importante dépolarisation réversible (73 ± 6 mV, n = 5) comme montré aux figures 4 b et 4c.

### Les propriétés unitaires du canal TWIK-1.

Les enregistrements de canal simple de courants, représentés à la figure 5, dans une configuration de patch de type "inside-out" ou dans une configuration de cellule entière montre que les canaux TWIK-1 assurent le passage de courants sortant ou entrant en fonction, respectivement, d'une dépolarisation ou d'une hyperpolarisation (figure 5a). La relation courant-voltage du canal unique, représentée à la figure 5b, montre une rectification entrante peu accentuée en présence de 3 mM (figure 5) et 10 mM (non représenté) de Mg²⁺ du coté cytoplasmique. Comme montré à la figure 5b, cette rectification disparaît en l'absence de Mg²⁺ interne. Dans 3 mM de Mg²⁺ interne, la durée moyenne d'ouverture à +80 mV est de 1,9 ms et la conductance unitaire de 19 ± 1 pS (figure 5c). A -80 mV, les canaux sont vacillants avec une durée moyenne d'ouverture de 0,3 ms, et une valeur de conductance en augmentation à 34 ± 4 pS. Le retrait des ions Mg²⁺ internes n'influence pas les paramètres cinétiques à la fois dans les conditions polarisées et dépolarisées, mais la conductance unitaire mesurée à -80 mV atteint 35 ± 4 pS. Cette augmentation apparente de la conductance dans le canal unique suggère que c'est la vacillance extrêmement rapide induite par Mg²⁺ qui conduit à une sous-estimation de la valeur réelle de la conductance. Les mêmes propriétés ont été observées dans la configuration de cellule fixée montrant que le comportement du canal n'est pas modifié par une excision du patch. Les canaux TWIK-1 dans des patch excisés ne se décharge pas et semble pas nécessité de constituants intra-cellulaires. Contrairement à de nombreux canaux qui nécessitent la présence d'ATP, pour leur activité dans la configuration de patch excisé, l'ATP n'est pas nécessaire à l'expression de TWIK-1. En outre, la perfusion du patch avec une solution contenant 10 mM d'ATP n'induit aucun effet sur l'activité du canal TWIK-1.

### Les propriétés de régulation de l'activité du canal TWIK-1.

Le pH intracellulaire (Phᵢ) est impliqué dans le contrôle de nombreux processus cellulaires, et dans des cellules commes les cellules hépatiques, les changement de Phᵢ régule le potentiel membranaire (Bear, C. E. et al., 1988, *Biochim. Biophys. Acta*, 944, 113-120).

L'acidification intracellulaire des oocytes a été obtenue selon deux méthodes :
- superfusion avec une solution enrichies en CO₂ qui produit une acidification par un mécanisme impliquant le système de transport de bicarbonate (Guillemare, E. et al., 1995, *Mol. Pharmacol.,* 47, 588-594);
- traitement avec le dinitrophénol (DNP), qui est un inhibiteur métabolique découplant le gradient de H⁺ dans les mitochondries et qui induit une acidité interne (Pedersen, P. L. et Carafoli, E., 1987, *Trends Biol. Sci*., 12, 146-189).

Ces deux expérimentations ont conduit à une réduction significative des courants de TWIK-1, supérieur à 95 % dans le cas de CO₂ et 80 % dans le cas du DNP, des valeurs de contrôle de l'amplitude, comme montré aux figures 6a à 6d. L'inhibition induite par le DNP sur l'activité du canal unique K⁺ a encore été observée dans des conditions de patch attaché, comme montré aux figures 6e à 6f. Toutefois, après excision du patch, l'activité du canal devient insensible à l'acidification de la solution interne obtenue, soit en modifiant le rapport de tampon Na₂HPO₄/NaH₂PO₄ (figures 6g et 6h) soit par bouillonnement de CO₂ (non représenté). EN conséquence, l'effet du pH sur l'activité du canal TWIK-1 est probablement indirect.

La phosphorylation ou la déphosphorylation de résidus d'acide aminés spécifiques est un important mécanisme de régulation des canaux ioniques (Levitan, I. B., 1994, *Annu. Rev. Physiol*., 56, 193-212). Comme montré à la figure 7, l'activation de la protéine kinase C par le phorbol-12 myristate acétate (PMA, 30 nM) augmente les courants de TWIK-1. Le phorbol ester 4α-phorbol-12, 13 didécanoate inactif (PDA, 1 µM) n'a acun effet. Dans un patch attaché qui initialement exprimait uniquement un canal, l'application du PMA la présence d'au moins cinq canaux (figures 7c et 7d). Cette expérience montre que au moins quatre canaux sont présents mais silencieux dans ce patch avant l'application de PMA. Du fait que la séquence de TWIK-1 contient un site de phosphorylation consensus pour la protéine kinase C (PKC), localisé au niveau de la thréonine en position 161 (figure 1b), l'effet du PMA laisse supposer une régulation sous le contrôle de PKC. Toutefois, la mutation de la thréonine 161 en alanine conduit à un cnal muté qui demeure fonctionnel et conserve la capacité d'être activé par le PMA.

L'activation de la protéine kinase A par application de 8-Cl-AMPc (300 µM) ou de forskolin (10 µM) n'affecte pas l'activité de TWIK-1. L'élévation de la concentration en Ca²⁺ cytoplasmique par application de A23187 (1 µM), qui pourrait avoir activé la Ca²⁺-calmoduline kinase II et/ou révélé la présence d'un canal activé par le Ca²⁺, est aussi sans effet sur les propriétés du canal TWIK-1.

La présente invention a donc pour objet une molécule d'acide nucléique isolée et purifiée codant pour une protéine constituant un canal potassium TWIK-1 ou présentant des propriétés et une structure du type de celles du canal TWIK-1 décrite ci-dessus.

Plus particulièrement, ladite molécule d'acide nucléique code pour la protéine TWIK-1 dont la séquence en acides aminés est représentée dans la liste de séquence en annexe sous le numéro SEQ ID NO:2, ou un dérivé fonctionnellement équivalent de cette séquence. De tels dérivés peuvent être obtenus en modifiant et/ou en supprimant un ou plusieurs résidus d'acides aminés de cette séquence, dès lors que cette modification et/ou supression ne modifie pas les propriétés fonctionnelles de canal potassium TWIK-1 de la protéine en résultant.

La séquence d'une molécule d'ADN codant pour cette protéine est plus particulièrement celle codant pour TWIK-1 représentée dans la liste de séquence en annexe sous le numéro SEQ ID NO:1.

L'invention concerne également un vecteur comprenant une molécule d'acide nucléique précédente, ainsi qu'un procédé de production ou d'expression dans un hôte cellulaire d'une protéine constituant un canal potassium TWIK-1 ou un canal de la même famille que TWIK-1.

Un procédé de production d'une protéine constituant un canal potassium TWIK-1 ou présentant des propriétés et une structure du type de celles du canal TWIK-1 consiste :
- à transférer une molécule d'acide nucléique de l'invention ou un vecteur contenant ladite molécule dans un hôte cellulaire,
- à cultiver l'hôte cellulaire obtenu à l'étape précédente dans des conditions permettant la production de canaux potassium présentant les propriétés de TWIK-1,
- à isoler, par tous moyens appropriés les protéines constituant des canaux potassium de la famille de TWIK-1.

Un procédé d'expression d'un canal potassium TWIK-1 ou de la même famille que TWIK-1 consiste :
- à transférer une molécule d'acide nucléique de l'invention ou un vecteur contenant ladite molécule dans un hôte cellulaire,
- à cultiver l'hôte cellulaire obtenu à l'étape précédente dans des conditions permettant l'expression de canaux potassium de la famille de TWIK-1.

L'hôte cellulaire mis en oeuvre dans les procédés précédents peut être choisi parmi les procaryotes ou les eucaryotes et notamment parmi les bactéries, les levures, les cellules de mammifères, de plantes ou d'insectes.

Le vecteur utilisé est choisi en fonction de l'hôte dans lequel il sera transféré, il peut s'agit de tout vecteur comme un plasmide.

L'invention concerne donc aussi les cellules transformées exprimant des canaux potassium présentant des propriétés et une structure du type de celles du canal TWIK-1 obtenues conformément aux procédés précédents.

Les cellules exprimant des canaux potassium TWIK-1 ou des canaux présentant des propriétés et une structure du type de celles du canal TWIK-1 obtenues conformément aux procédés, précédents, sont utiles pour le criblage de substances capables de moduler l'activité des canaux potassium TWIK-1. Ce criblage est effectué en mettant en contact des quantités variables d'une substance à tester avec des cellules exprimant le canal TWIK-1 ou des canaux potassium présentant des propriétés et une structure du type de celles du canal TWIK-1, puis en mesurant, par tous moyens appropriés, les effets éventuels de ladite substance sur les courants des canaux potassium de ces canaux.

Ce procédé de criblage permet d'identifier des drogues utiles dans le traitement des maladies du coeur ou du système nerveux. Des pathologies impliquant les canaux potassium et donc susceptible de concerner les canaux de la famille de TWIK-1 sont par exemple, l'épilepsie, les pathologies cardiaques (arythmies) et vasculaires, les neurodégénérescences particulièrement celles qui sont associées aux ischémies ou anoxies, les pathologies endocriniennes associées à des anomalies de sécrétion d'hormones, les pathologies musculaires.

Une molécule d'acide nucléique isolée et purifiée codant pour une protéine constituant un canal potassium TWIK-1 ou un vecteur comprenant cette molécule d'acide nucléique ou encore une cellule exprimant des canaux potassium TWIK-1, sont aussi utiles pour la préparation d'animaux transgéniques NON-HUMAIN. Il peut s'agir d'animaux sur-exprimant lesdits canaux, mais surtout d'animaux dit "knock out", c'est à dire présentant une déficience en ces canaux; ces animaux transgéniques NON-HUMAIN sont préparés par des méthodes connus de l'homme du métier, et permettent de disposer de modèles vivants pour l'étude de pathologies animales associées aux canaux TWIK-1.

Les molécules d'acide nucléique de l'invention ou les cellules transformées par ladite molécule sont en outre susceptibles d'être utilisées dans des stratégies de thérapie génique afin de compenser une déficience des canaux potassium au niveau de un ou plusieurs tissus d'un patient. L'invention concerne donc aussi un médicament comprenant des molécules d'acide nucléique de l'invention ou de cellules transformées par ladite molécule pour le traitement de pathologie impliquant les canaux potassium.

En outre, le gène du canal TWIK-1 a été situé sur le chromosome 1 en position q42-q43. La localisation chromosomique de ce gène constitue un résultat déterminant pour l'identification de maladies génétiques associées à cette nouvelle famille de canaux potassium; ainsi, la connaissance de la structure des canaux de la famille de TWIK-1 est de nature à permettre la réalisation d'un diagnostic ante-natal de telles maladies.

La présente invention a encore pour objet une nouvelle famille de canaux K⁺, à laquelle appartient TWIK-1, présents dans la plupart des tissus humains et plus particulièrement abondant dans le cerveau et le coeur, et présentant des propriétés et une structure du type de celles du canal TWIK-1 décrites ci-dessus. Elle concerne donc une protéine isolée et purifiée dont la séquence en acides aminés est représentée dans la liste de séquence en annexe sous le numéro SEQ ID NO:2, ou un dérivé fonctionnellement équivalent de cette séquence.

De tels dérivés peuvent être obtenus en modifiant et/ou en supprimant un ou plusieurs résidus d'acides aminés de cette séquence ou en segmentant cette séquence, dès lors que cette modification et/ou supression ou délétion d'un fragment ne modifie pas les propriétés fonctionnelles de canal potassium du type TWIK-1, de la protéine en résultant.

Une protéine constituant un canal potassium du type TWIK-1 est utile pour la fabrication de médicaments destinés à traiter ou prévenir des maladies impliquant un dysfonctionnement des canaux potassium.

Des anticorps poly ou mono-clonaux dirigés contre une protéine constituant un canal potassium du type TWIK-1 peuvent être préparés par les méthodes classiques décrites dans la littérature.

Ces anticorps sont utiles pour rechercher la présence de canaux potassium de la famille de TWIK-1 dans différentes tissus humains ou naimaux, mais ils peuvent aussi trouver des applications dans le domaine thérapeutique pour inhiber ou activer *in vivo,* grâce à leur spécificité, les canaux potassium du type TWIK-1.

D'autres avantages et caractéristiques de l'invention apparaîtront à la lecture des exemples qui suivent donnés à titre non limitatifs et concernant le clonage et l'expression de TWIK-1.

### Identification de la séquence HSC3AH031 EST et analyse de l'ARN.

Les domaines P des canaux clonés ont été utilisés pour rechercher des homolhues dans des bases de données du NCBI (National Center of Biotechnology) en mettant en oeuvre le programme d'alignement de séquences tBLASTn. La traduction d'une séquence EST (HSC3AH031, numéro d'accès Genbank : F12504) présentait une similarité de séquence significative (P = 1,2 x 10⁻³) avec le second domaine P d'un canal K⁺ de levure. Cette séquence de 298 pb a été originellement obtenue à partir d'une banque d'ADNc de cerveau humain dans le cadre du programme d'ADNc Genexpress (Auffray, C. et al., 1995, *C. R. Acad. Sci., III, Sci. Vie,* 318, 263-272). Un fragment d'ADN de 255 pb correspondant à HSC3AH031 a été amplifié par PCR à partir d'ADNc dérivés de poly(A)⁺ de cerveau humain et sous-cloné dans pBluescript (Stratagene) pour donner pBS-HSC3A.

Pour l'analyse d'ARN, un Nothern blot de tissus humains multiples (Clontech) a été criblé avec l'insert de pBS-HSCA marqué au P³² dans 50 % de formamide, 5 x SSPE (0,9 M NaCl ; 50 mM phosphate de sodium ; pH 7,4 ; 5 mM EDTA), 0,1 % SDS, 5 x Denhardts, 20 mM phosphate de potassium, pH 6,5 et 250 µg d'ADN de sperme de saumon dénaturé à 55 °C pendant 18 heures. Les blots ont été lavés jusqu'à une stringence finale de 0,1 SSC (3 M NaCl ; 0,3 M citrate de sodium ; pH 7,0), 0,3 % SDS à 65 °C.

### Isolement du clone d'ADNC codant TWIK-1.

Une banque d'ADNc oligo(dT) issu d'ARN poly(A)⁺ isolés de rein d'humain adulte a été criblée avec l'insert de pBS-HSCA marqué au P³². Les filtres ont été hybridés dans 50 % formamide, 5 x SSC, 4 X Denhardt, 0,1 % SDS et 100 µg d'ADN de sperme de saumon dénaturé à 50 °C pendant 18 heures. Quatre cloes dhybridation positive ont été isolés d'environ 5 x 10⁵ clones. Les phages λZAPII contenant les inserts d'ADNc ont été convertis en plasmides d'ADNc (Stratagene). Les inserts d'ADNc ont été caractérisés en analyse par des enzymes de restriction et par séquençage total ou partiel d'ADN sur les deux brins par la méthode des nucléotide didéoxy mettant en oeuvre un séquenceur automatique (Applied Biosystems 373A).

### Mutations, synthèse d'ARNc et injection d'oocyte.

La séquence codante de TWIK-1 a été amplifiée en utilisant une ADN polymerase à faible taux d'erreur (Pwo DNA pol, Boehringer) et sous-clonée dans le plasmide pEXO pour donner le pEXO-TWIK-1. Les mutations ont été réalisées en amplifiant le plasmide pEXO-TWIK-1 en entier avec un kit PCR d'extension hautement fidèle (Boehringer) et deux amorces adjacentes. L'un d'entre-eux a introduit une mutation ponctuelle dans la séquence codante de TWIK-1 changant le codon de la Thr 161 en un codon pour l'alanine. Le produit de la PCR a été linéarisé par l'enzyme BamHI et les ARNc ont été synthétisés en utilisant une T7 ARN polymérase (Stratagene). La préparation des oocytes de *X. laevis* et l'injection d'ARNC ont été réalisées comme décrit dans la littérature (Guillemare, E. et al., 1992, *Biochemistry*, 31, 12463-12468).

### Mesures électrophysiologique.

Dans une chambre à perfusion de 0,3 ml, un seul oocytea été empalé sur deux micro-électrodes de verre standards (0,5 - 2,0 MW) chargées avec 3 M KCl et maintenues sous voltage-clamp avec un amplificateur Dagan TEV200. La solution du bain contenait 98 mM KCl, 1,8 mM CaCl₂, 2 mM MgCl₂ et 5 mM HEPES à pH 7,4 avec KOH. La stimulation de la préparation, l'acquisition des données et les analyses ont été réalisées avec le logiciel pClamp (Axon Instruments, USA).

Pour les expériences de patch-clamp, les oocytes ont été débarassés de leur membrane vitelline comme décrit dans la littérature (Duprat, F. et al., 1995, *Biochem. Biophys. Res. Commun.,* 212, 657-663) et placés'dans une solution de bain contenant 140 mM KCl, 1,8 mM CaCl₂, 2 mM MgCl₂ et 5 mM HEPES à pH 7,4 avec KOH. Les pipettes ont été remplies avec une solution forte de K⁺ (40 mM KCL, 100 mM de sulphonate méthane de potassium, 1,8 mM CaCl₂, 2 mM MgCl₂ et 5 mM HEPES ajusté à pH 7,4 avec KOH). 100 µM de GdCl₃ ont été ajoutés à la solution de pipette pour inhiber l'action des canaux activés. Les patch "inside-out" ont été perfusés avec une solution contenant 140 mM KCL, 10 mM CaCl₂, 5 mM HEPES ajusté à pH 7,2 avec KOH et 5 mM EGTA ajoutés journellement. Les signaux de canal unique ont été filtrés à 3,5 kHz et analysés avec le programme Biopatch (Bio-Logic, Grnoble, France)

### LISTE DE SÉQUENCES.

### INFORMATION CONCERNANT LA SEQ ID NO:1 :

I - CARACTRERISTIQUE DE LA SEQUENCE :
   A) LONGUEUR :
   B) TYPE :
   C) NOMBRE DE BRIN :
   D) CONFIGURATION :
II - TYPE DE MOLECULE :
XI - DESCRIPTION DE SEQUENCE : SEQ ID NO:1 :

GGGCAGGAAG ACGGCGCTGC CCGGAGGAGC GGGGCGGGCG GGCGCGCGGG GGAGCGGGCG 60

GCGGGCGGGA GCCAGGCCCG GGCGGGGGCG GGGGCGGCGG GGCCAGAAGA GGCGGCGGGC 120

CGCGCTCCGG CCGGTCTGCG GCGTTGGCCT TGGCTTTGGC TTTGGCGGCG GCGGTGGAGA 180

AG ATG CTG CAG TCC CTG GCC GGC AGC TCG TGC GTG CGC CTG GTG GAG CGG 230
Met Leu Gln Ser Leu Ala Gly Ser Ser Cys Val Arg Leu Val Glu Arg
1               5                   10                  15

CAC CGC TCG GCC TGG TGC TTC GGC TTC CTG GTG CTG GGC TAC TTG CTC 278
His Arg Ser Ala Trp Cys Phe Gly Phe Leu Val Leu Gly Tyr Leu Leu
            20                  25                  30

TAC CTG GTC TTC GGC GCA GTG GTC TTC TCC TCG GTG GAG CTG CCC TAT 326
Tyr Leu Val Phe Gly Ala Val Val Phe Ser Ser Val Glu Leu Pro Tyr
        35                  40                  45

GAG GAC CTG CTG CGC CAG GAG CTG CGC AAG CTG AAG CGA CGC TTC TTG 374
Glu Asp Leu Leu Arg Gln Glu Leu Arg Lys Leu Lys Arg Arg Phe Leu
    50                  55                  60

GAG GAG CAC GAG TGC CTG TCT GAG CAG CAG CTG GAG CAG TTC CTG GGC 422
Glu Glu His Glu Cys Leu Ser Glu Gln Gln Leu Glu Gln Phe Leu Gly
65                  70                  75                  80

CGG GTG CTG GAG GCC AGC AAC TAC GGC GTG TCG GTG CTC AGC AAC GCC 470
Arg Val Leu Glu Ala Ser Asn Tyr Gly Val Ser Val Leu Ser Asn Ala
                85                  90                  95

TCG GGC AAC TGG AAC TGG GAC TTC ACC TCC GCG CTC TTC TTC GCC AGC 518
Ser Gly Asn Trp Asn Trp Asp Phe Thr Ser Ala Leu Phe Phe Ala Ser
            100                 105                 110

ACC GTG CTC TCC ACC ACA GGT TAT GGC CAC ACC GTG CCC TTG TCA GAT 566
Thr Val Leu Ser Thr Thr Gly Tyr Gly His Thr Val Pro Leu Ser Asp
        115                 120                 125

GGA GGT AAG GCC TTC TGC ATC ATC TAC TCC GTC ATT GGC ATT CCC TTC 614
Gly Gly Lys Ala Phe Cys Ile Ile Tyr Ser Val Ile Gly Ile Pro Phe
    130                 135                 140

ACC CTC CTG TTC CTG ACG GCT GTG GTC CAG CGC ATC ACC GTG CAC GTC 662
Thr Leu Leu Phe Leu Thr Ala Val Val Gln Arg Ile Thr Val His Val
145                 150                 155                 160

ACC CGC AGG CCG GTC CTC TAC TTC CAC ATC CGC TGG GGC TTC TCC AAG 710
Thr Arg Arg Pro Val Leu Tyr Phe His Ile Arg Trp Gly Phe Ser Lys
                165                 170                 175

CAG GTG GTG GCC ATC GTC CAT GCC GTG CTC CTT GGG TTT GTC ACT GTG 758
Gln Val Val Ala Ile Val His Ala Val Leu Leu Gly Phe Val Thr Val
            180                 185                 190

TCC TGC TTC TTC TTC ATC CCG GCC GCT GTC TTC TCA GTC CTG GAG GAT 806
Ser Cys Phe Phe Phe Ile Pro Ala Ala Val Phe Ser Val Leu Glu Asp
        195                 200                 205

GAC TGG AAC TTC CTG GAA TCC TTT TAT TTT TGT TTT ATT TCC CTG AGC 854
Asp Trp Asn Phe Leu Glu Ser Phe Tyr Phe Cys Phe Ile Ser Leu Ser
    210                 215                 220

ACC ATT GGC CTG GGG GAT TAT GTG CCT GGG GAA GGC TAC AAT CAA AAA 902
Thr Ile Gly Leu Gly Asp Tyr Val Pro Gly Glu Gly Tyr Asn Gln Lys
225                 230                 235                 240

TTC AGA GAG CTC TAT AAG ATT GGG ATC ACG TGT TAC CTG CTA CTT GGC 950
Phe Arg Glu Leu Tyr Lys Ile Gly Ile Thr Cys Tyr Leu Leu Leu Gly
                245                 250                 255

CTT ATT GCC ATG TTG GTA GTT CTG GAA ACC TTC TGT GAA CTC CAT GAG 998
Leu Ile Ala Met Leu Val Val Leu Glu Thr Phe Cys Glu Leu His Glu
            260                 265                 270

CTG AAA AAA TTC AGA AAA ATG TTC TAT GTG AAG AAG GAC AAG GAC GAG 1046
Leu Lys Lys Phe Arg Lys Met Phe Tyr Val Lys Lys Asp Lys Asp Glu
        275                 280                 285

GAT CAG GTG CAC ATC ATA GAG CAT GAC CAA CTG TCC TTC TCC TCG ATC 1094
Asp Gln Val His Ile Ile Glu His Asp Gln Leu Ser Phe Ser Ser Ile
    290                 295                 300

ACA GAC CAG GCA GCT GGC ATG AAA GAG GAC CAG AAG CAA AAT GAG CCT 1142
Thr Asp Gln Ala Ala Gly Met Lys Glu Asp Gln Lys Gln Asn Glu Pro
305                 310                 315                 320

TTT GTG GCC ACC CAG TCA TCT GCC TGC GTG GAT GGC CCT GCA AAC CAT 1190
Phe Val Ala Thr Gln Ser Ser Ala Cys Val Asp Gly Pro Ala Asn His
325 330 336

TGA GCGTAGGATT TGTTGCATTA TGCTAGAGCA CCAGGGTCAG GGTGCAAGGA 1243
*

AGAGGCTTAA GTATGTTCAT TTTTATCAGA ATGCAAAAGC GAAAATTATG TCACTTTAAG 1303

AAATAGCTAC TGTTTGCAAT GTCTTATTAA AAAACAACAA AAAAAGACAC ATGGAACAAA 1363

GAAGCTGTGA CCCCAGCAGG ATGTCTAATA TGTGAGGAAA TGAGATGTCC ACCTAAAATT 1423

CATATGTGAC AAAATTATCT CGACCTTACA TAGGAGGAGA ATACTTGAAG CAGTATGCTG 1483

CTGTGGTTAG AAGCAGATTT TATACTTTTA ACTGGAAACT TTGGGGTTTG CATTTAGATC 1543

ATTTAGCTGA TGGCTAAATA GCAAAATTTA TATTTAGAAG CAAAAAAAAA AAGCATAGAG 1603

ATGTGTTTTA TAAATAGGTT TATGTGTACT GGTTTGCATG TACCCACCCA AAATGATTAT 1663
TTTTGGAGAA TCTAAGTCAA ACTCACTATT TATAATGCAT AGGTAACCAT TAACTATGTA 1723

CATATAAAGT ATAAATATGT TTATATTCTG TACATATGGT TTAGGTCACC AGATCCTAGT 1783

GTAGTTCTGA AACTAAGACT ATAGATATTT TGTTTCTTTT GATTTCTCTT TATACTAAAG 1843

AATCCAGAGT TGCTACAATA AAATAAGGGG AATAATAAAA AAAAAAAAAA A 1894

### INFORMATION CONCERNANT LA SEQ ID NO :2

I - CARACTRERISTIQUE DE LA SEQUENCE :
   A) LONGUEUR :
   B) TYPE :
   C) NOMBRE DE BRIN :
   D) CONFIGURATION :
II - TYPE DE MOLECULE :
XI - DESCRIPTION DE LA SEQUENCE : SEQ ID NO:2 :

Met Leu Gln Ser Leu Ala Gly Ser Ser Cys Val Arg Leu Val Glu Arg
1               5                   10                  15

His Arg Ser Ala Trp Cys Phe Gly Phe Leu Val Leu Gly Tyr Leu Leu
            20                  25                  30

Tyr Leu Val Phe Gly Ala Val Val Phe Ser Ser Val Glu Leu Pro Tyr
        35                  40                  45

Glu Asp Leu Leu Arg Gln Glu Leu Arg Lys Leu Lys Arg Arg Phe Leu
    50                  55                  60

Glu Glu His Glu Cys Leu Ser Glu Gln Gln Leu Glu Gln Phe Leu Gly
65                  70                  75                  80

Arg Val Leu Glu Ala Ser Asn Tyr Gly Val Ser Val Leu Ser Asn Ala
                85                  90                  95

Ser Gly Asn Trp Asn Trp Asp Phe Thr Ser Ala Leu Phe Phe Ala Ser
            100                 105                 110

Thr Val Leu Ser Thr Thr Gly Tyr Gly His Thr Val Pro Leu Ser Asp
        115                 120                 125

Gly Gly Lys Ala Phe Cys Ile Ile Tyr Ser Val Ile Gly Ile Pro Phe
    130                 135                 140

Thr Leu Leu Phe Leu Thr Ala Val Val Gln Arg Ile Thr Val His Val
145                 150                 155                 160

Thr Arg Arg Pro Val Leu Tyr Phe His Ile Arg Trp Gly Phe Ser Lys
                165                 170                 175

Gln Val Val Ala Ile Val His Ala Val Leu Leu Gly Phe Val Thr Val
            180                 185                 190

Ser Cys Phe Phe Phe Ile Pro Ala Ala Val Phe Ser Val Leu Glu Asp
        195                 200                 205

Asp Trp Asn Phe Leu Glu Ser Phe Tyr Phe Cys Phe Ile Ser Leu Ser
    210                 215                 220

Thr Ile Gly Leu Gly Asp Tyr Val Pro Gly Glu Gly Tyr Asn Gln Lys
225                 230                 235                 240

Phe Arg Glu Leu Tyr Lys Ile Gly Ile Thr Cys Tyr Leu Leu Leu Gly
                245                 250                 255

Leu Ile Ala Met Leu Val Val Leu Glu Thr Phe Cys Glu Leu His Glu
            260                 265                 270

Leu Lys Lys Phe Arg Lys Met Phe Tyr Val Lys Lys Asp Lys Asp Glu
        275                 280                 285

Asp Gln Val His Ile Ile Glu His Asp Gln Leu Ser Phe Ser Ser Ile
    290                 395                 300

Thr Asp Gln Ala Ala Gly Met Lys Glu Asp Gln Lys Gln Asn Glu Pro
305                 310                 315                 320

Phe Val Ala Thr Gln Ser Ser Ala Cys Val Asp Gly Pro Ala Asn His
                325                 330                         336

## Revendications

1. Molécule d'acide nucléique isolée et purifiée codant pour une protéine constituant un canal potassium à quatre segments transmembranaires et deux domaines P et présentant des propriétés de rectification entrante peu marquées.

2. Molécule d'acide nucléique isolée et purifiée codant pour une protéine constituant un canal potassium, **caractérisée** en ce quelle code pour la protéine dont la séquence en acides aminés est représentée dans la liste de séquence en annexe sous le numéro SEQ ID NO:2.

3. Molécule d'acide nucléique selon la revendication 2, dont la séquence est représentée dans la liste de séquence en annexe sous le numéro SEQ ID NO:1.

4. Vecteur comprenant une molécule d'acide nucléique selon l'une quelconque des revendications 1 à 3.

5. Procédé de production d'une protéine constituant un canal potassium à quatre segments transmembranaires et deux domaines P et présentant des propriétés de rectification entrante peu marquées consistant :
- à transférer une molécule d'acide nucléique selon l'une quelconque des revendications 1 à 3 ou un vecteur selon la revendication 4, dans un hôte cellulaire,
- à cultiver, *in vitro*, l'hôte cellulaire obtenu à l'étape précédente dans des conditions permettant la production de canaux potassium à quatre segments transmembranaires et deux domaines P et présentant des propriétés de rectification entrante peu marquées,
- à isoler, par tous moyens appropriés les protéines constituant des canaux potassium à quatre segments transmembranaires et deux domaines P et présentant des propriétés de rectification entrante peu marquées.

6. Procédé d'expression d'un canal potassium à quatre segments transmembranaires et deux domaines P et présentant des propriétés de rectification entrante peu marquées consistant :
- à transférer une molécule d'acide nucléique selon l'une quelconque des revendications 1 à 3 ou un vecteur selon la revendication 4, dans un hôte cellulaire,
- à cultiver, *in vitro*, l'hôte cellulaire obtenu à l'étape précédente dans des conditions permettant l'expression de canaux potassium à quatre segments transmembranaires et deux domaines P et présentant des propriétés de rectification entrante peu marquées.

7. Procédé selon l'une quelconque des revendications 5 et 6, **caractérisé en ce que** l'hôte cellulaire est choisi parmi les procaryotes ou les eucaryotes et notamment parmi les bactéries, les levures, les cellules de mammifères, de plantes ou d'insectes.

8. Cellule transformée exprimant des canaux potassium à quatre segments transmembranaires et deux domaines P et présentant des propriétés de rectification entrante peu marquées **caractérisée en ce qu'**une molécule d'acide nucléique selon l'une quelconque des revendications 1 à 3 ou un vecteur selon la revendication 4 ont été transférés dans ladite cellule.

9. Procédé de criblage de substances capables de moduler l'activité des canaux potassium à quatre segments transmembranaires et deux domaines P et présentant des propriétés de rectification entrante peu marquées, **caractérisé en ce que** :
- l'on met en contact des quantités variables d'une substance à tester avec des cellules exprimant des canaux potassium à quatre segments transmembranaires et deux domaines P et présentant des propriétés de rectification entrante peu marquées selon la revendication 8 , puis
- l'on mesure, par tous moyens appropriés, les effets éventuels de ladite substance sur les courants des canaux potassium à quatre segments transmembranaires et deux domaines P et présentant des propriétés de rectification entrante peu marquées.

10. Composition pharmaceutique pour compenser une déficience des canaux potassium au niveau de un ou plusieurs tissus, **caractérisée** en en ce qu'elle comprend des molécules d'acide nucléique selon l'une quelconque des revendications 1 à 3, ou un vecteur selon la revendication 4, ou encore des cellules selon la revendication 8.

11. Protéine isolée et purifiée constituant un canal potassium à quatre segments transmembranaires et deux domaines P et présentant des propriétés de rectification entrante peu marquées.

12. Protéine selon la revendication 11 dont la séquence en acides aminés est représentée dans la liste de séquence en annexe sous le numéro SEQ ID NO:2.

13. Composition pharmaceutique pour compenser une déficience des canaux potassium au niveau de un ou plusieurs tissus, **caractérisée** en en ce quelle comprend une protéine selon l'une quelconque des revendications 11 et 12.

14. Anticorps monoclonal ou polyclonal dirigé contre une protéine selon l'une quelconque des revendications 11 et 12.

## Patentansprüche

1. Isoliertes und gereinigtes codierendes Nukleinsäuremolekül für ein Protein, welches einen Kaliumkanal mit vier transmembranen Segmenten und zwei P-Domainen bildet, und welcher schwach ausgeprägte Eigenschaften für die eintretende Rektifikation aufweist.

2. Isoliertes und gereinigtes codierendes Nukleinsäuremolekül für ein Protein, welches einen Kaliumkanal bildet, **dadurch gekennzeichnet, dass** es das Protein, dessen Sequenz an Aminosäuren in der Liste der Sequenzen des Annex unter der Nummer SEQ ID NR. 2 angegeben ist, codiert.

3. Nukleinsäuremolekül nach Anspruch 2, dessen Sequenz in der Liste der Sequenzen des Annex unter der Nummer SEQ ID NR. 1 angegeben ist.

4. Vektor, welcher ein Nukleinsäuremolekül nach einem der Ansprüche 1 bis 3 enthält.

5. Produktionsverfahren eines Proteins, welches einen Kaliumkanal mit vier transmembranen Segmenten und zwei P-Domainen bildet, und welcher schwach ausgeprägte Eigenschaften für die eintretende Rektifikation aufweist, bestehend aus:
- ein Nukleinsäuremolekül nach einem der Ansprüche 1 bis 3 oder einen Vektor nach Anspruch 4 in einen zellulären Wirt zu transferieren,
- den zellulären Wirt *in vitro* zu kultivieren, welcher in der vorhergehenden Etappe, deren Konditionen die Herstellung der Kaliumkanäle mit vier transmembranen Segmenten und zwei P-Domainen, und welche schwach ausgeprägte Eigenschaften für die eintretende Rektifikation aufweisen, ermöglichen, gewonnen wurde,
- die Proteine mit allen Mitteln zu isolieren, welche den Proteinen, welche aus Kaliumkanälen mit vier transmembranen Segmenten und zwei P-Domainen bestehen, und welche schwach ausgeprägte Eigenschaften für die eintretende Rektifikation aufweisen, angepasst sind.

6. Verfahren zur Expression eines Kaliumkanals mit vier transmembranen Segmenten und zwei P-Domainen, und welcher schwach ausgeprägte Eigenschaften für die eintretende Rektifikation aufweist, bestehend aus:
- ein Nukleinsäuremolekül nach einem der Ansprüche 1 bis 3 oder einen Vektor nach Anspruch 4 in einen zellulären Wirt zu transferieren,
- den zellulären Wirt *in vitro* zu kultivieren, welcher in der vorhergehenden Etappe, deren Konditionen die Expression der Kaliumkanäle mit vier transmembranen Segmenten und zwei P-Domainen, und welche schwach ausgeprägte Eigenschaften für die eintretende Rektifikation aufweisen, ermöglichen, gewonnen wurde.

7. Verfahren nach einem der Ansprüche 5 und 6, **dadurch gekennzeichnet, dass** der zelluläre Wirt ausgewählt wurde unter den Prokaryoten oder den Eukaryoten und vor allem unter den Bakterien, den Hefen, den Säugetierzellen, den Pflanzen oder den Insekten.

8. Transformierte Zelle, welche die Kaliumkanäle mit vier transmembranen Segmenten und zwei P-Domainen, und welche schwach ausgeprägte Eigenschaften für die eintretende Rektifikation aufweisen, ausdrückt, **dadurch gekennzeichnet, dass** ein Nukleinsäuremolekül nach einem der Ansprüche 1 bis 3 oder ein Vektor nach Anspruch 4 in die besagte Zelle transferiert wurde.

9. Verfahren für das Screening von Substanzen, welche in der Lage sind die Aktivität der Kaliumkanäle mit vier transmembranen Segmenten und zwei P-Domainen, und welche schwach ausgeprägte Eigenschaften für die eintretende Rektifikation aufweisen, zu verändern, **dadurch gekennzeichnet, dass**:
- man variable Quantitäten der zu testenden Substanz mit den Zellen nach Anspruch 8, welche die Kaliumkanäle mit vier transmembranen Segmenten und zwei P-Domainen, und welche schwach ausgeprägte Eigenschaften für die eintretende Rektifikation aufweisen, ausdrücken, in Kontakt bringt, anschließend
- man mit allen angepassten Mitteln, die eventuellen Effekte der besagten Substanz auf die Strömung der Kaliumkanäle mit vier transmembranen Segmenten und zwei P-Domainen, und welche schwach ausgeprägte Eigenschaften für die eintretende Rektifikation aufweisen, misst.

10. Pharmazeutische Zusammensetzung zur Kompen-sierung eines Defizits der Kaliumkanäle auf dem Niveau eines oder mehrerer Gewebe, **dadurch gekennzeichnet, dass** sie die Nukleinsäuremoleküle nach einem der Ansprüche 1 bis 3 oder auch die Zellen nach Anspruch 8 beinhaltet.

11. Isoliertes und gereinigtes Protein, welches einen Kaliumkanal mit vier transmembranen Segmenten und zwei P-Domainen, und welcher schwach ausgeprägte Eigenschaften für die eintretende Rektifikation aufweist, bildet.

12. Protein nach Anspruch 11, dessen Aminosäuresequenz in der Liste der Sequenzen des Annex unter der Nummer SEQ ID NR. 2 repräsentiert ist.

13. Pharmazeutische Zusammensetzung zur Kompen-sierung eines Defizits der Kaliumkanäle auf dem Niveau eines oder mehrerer Gewebe, **dadurch gekennzeichnet, dass** sie ein Protein nach einem der Ansprüche 11 und 12 beinhaltet.

14. Monoklonaler oder polyklonaler Antikörper, welcher gegen ein Protein nach einem der Ansprüche 11 und 12 gerichtet ist.

## Claims

1. Isolated and purified nucleic acid molecule coding for a protein forming a potassium channel with four transmembrane segments and two P domains and with weakly inward rectification properties.

2. Isolated and purified nucleic acid molecule coding for a protein forming a potassium channel, **characterised in that** it codes for the protein for which the amino acid sequence is represented in the sequence listing in the appendix under the number SEQ ID NO: 2.

3. Nucleic acid molecule according to claim 2, for which the sequence is represented in the sequence listing in the appendix under the number SEQ ID NO: 1.

4. Vector comprising a nucleic acid molecule according to any one of claims 1 to 3.

5. Method to produce a protein forming a potassium channel with four transmembrane segments and two P domains and with weakly inward rectification properties consisting of:
- transferring a nucleic acid molecule according to any one of claims 1 to 3 or a vector according to claim 4, into a cell host,
- cultivating the cell host obtained in the previous step, in vitro, under conditions enabling the production of potassium channels with four transmembrane segments and two P domains and with weakly inward rectification properties,
- using any appropriate means to isolate proteins forming potassium channels with four transmembrane segments and two P domains and with weakly inward rectification properties.

6. Method to express a potassium channel with four transmembrane segments and two P domains and with weakly inward rectification properties consisting of:
- transferring a nucleic acid molecule according to any one of claims 1 to 3 or a vector according to claim 4, into a cell host,
- cultivating the cell host obtained in the previous step, in vitro, under conditions enabling the expression of potassium channels with four transmembrane segments and two P domains and with weakly inward rectification properties,

7. Method according to either of claims 5 and 6, **characterised in that** the cell host is chosen from among procaryotes and eucaryotes, particularly among bacteria, yeasts, mammal cells, plant cells or insect cells.

8. Transformed cell expressing potassium channels with four transmembrane segments and two P domains and with weakly inward rectification properties, **characterised in that** a nucleic acid molecule according to any one of claims 1 to 3 or a vector according to claim 4 have been transferred into said cell.

9. Screening method for substances capable of modulating the activity of potassium channels with four transmembrane segments and two P domains and with weakly inward rectification properties, **characterised in that**:
- variable quantities of a substance to be tested are brought into contact with cells expressing potassium channels with four transmembrane segments and two P domains and with weakly inward rectification properties according to claim 8, and then
- any appropriate means are used to measure the effects of said substance on the currents in the potassium channels with four transmembrane segments and two P domains and with weakly inward rectification properties.

10. Pharmaceutical composition to compensate for a deficiency of potassium channels in one or more tissues, **characterised in that** it comprises nucleic acid molecules according to any one of claims 1 to 3, or a vector according to claim 4, or cells according to claim 8.

11. Isolated and purified protein forming a potassium channel with four transmembrane segments and two P domains and with weakly inward rectification properties.

12. Protein according to claim 11, for which the amino acid sequence is represented in the sequence listing in the appendix under the number SEQ ID NO: 2.

13. Pharmaceutical composition to compensate for a deficiency in potassium channels in one or several tissues, **characterised in that** it comprises a protein according to either of claims 11 and 12.

14. Monoclonal or polyclonal antibody directed against a protein according to either of claims 11 and 12.
